# EUROPÄISCHE PATENTSCHRIFT

(11) **EP 2 058 021 B1**
(45) Veröffentlichungstag und Bekanntmachung des Hinweises auf die Patenterteilung: **27.07.2016**
(21) Anmeldenummer: 09152374.6
(22) Anmeldetag: 11.01.2006
(51) Int. Cl.: A61M 5/315, A61M 5/24

(54) **Verabreichungsvorrichtung mit Anzeigetrommel**
Administration device comprising a display drum
Dispositif d'administration comportant un tambour d'affichage

(30) Priorität: 23.02.2005 DE 102005008280
(43) Veröffentlichungstag der Anmeldung: 13.05.2009
(62) Teilanmeldung aus: 06700025.7
(73) Patentinhaber: Tecpharma Licensing AG, 3401 Burgdorf (CH)
(72) Erfinder: Kirchhofer, Fritz, 3454, Sumiswald (CH)
(74) Vertreter: Schwabe - Sandmair - Marx

(56) Entgegenhaltungen:
- EP-A2- 0 937 476
- WO-A-99/38554
- WO-A1-2005/046770
- US-A1- 2002 052 578

## Beschreibung

Die Erfindung betrifft eine Vorrichtung für die Verabreichung eines fluiden Produkts, das insbesondere ein Medikament, ein Diagnosemittel oder ein kosmetisches Produkt sein kann. Bevorzugt wird die Erfindung in der Selbstverabreichung eingesetzt, obgleich sie hierauf nicht beschränkt ist, sondern auch in der Verabreichung durch medizinisch geschultes Personal zum Einsatz gelangen kann.

In Therapien, in denen medizinisch nicht geschulte Personen einen Wirkstoff verabreichen, beispielsweise sich selbst, müssen die verwendeten Verabreichungsvorrichtungen einfach, aber dennoch sicher handhabbar sein. Insbesondere muss sichergestellt sein, dass bei Vorrichtungen, die eine Einstellung der zu verabreichenden Produktdosis erlauben, die eingestellte Dosis zweifelfrei angezeigt wird. In der Diabetestherapie, einem bevorzugten Verwendungsfeld der Erfindung, ist auch zu berücksichtigen, dass die sich das Insulin selbst verabreichenden Personen sehgeschädigt sein können und die eingestellte Dosis daher besonders deutlich und eindeutig auf einer Dosisskala ablesbar sein sollte. Der guten Ablesbarkeit zuträglich ist es, wenn die Dosisskala auf einer Anzeigetrommel aufgetragen ist, die rotatorisch und translatorisch bewegbar ist, da die Dosisskala in diesem Fall längs und in Drehrichtung der Anzeigetrommel erstreckt werden kann.

Vorrichtungen der genannten Art sind beispielsweise aus der WO 99/38554 A1 bekannt. Die Vorrichtungen sind komplex und benötigen Verdrehsicherungen, die eine Drehbewegung einer Kolbenstange in die eine Drehrichtung zulassen, aber in die andere verhindern und zusätzlich auf zu übertragende oder gerade noch übertragbare Drehmomente abgestimmt sein müssen.

Aus der WO 03/075985 A1 ist eine einfachere Vorrichtung der genannten Art bekannt. Wie bei den Vorrichtungen der WO 99/38554 bedarf es allerdings zweier Spindeltriebe, die beide nicht selbsthemmend gebildet sind, so dass bei Einstellung einer nur kleinen Dosis auch nur ein kurzer Hub der Anzeigetrommel zur Verfügung steht.

Es ist eine Aufgabe der Erfindung, eine Verabreichungsvorrichtung der genannten Art, d.h. eine Verabreichungsvorrichtung mit einer Anzeigetrommel und Spindeltrieben zu schaffen, die einfach aufgebaut ist und daher preiswert angeboten werden kann, aber dennoch in Bezug auf eine Übersetzung oder gegebenenfalls auch Untersetzung zwischen einem Ausschütthub einer Fördereinrichtung und einer Verstellbewegung der Anzeigetrommel bei der Einstellung der Produktdosis an die Bedürfnisse der Verabreichung flexibel anpassbar ist.

Die Aufgabe der Erfindung wird durch die Verabreichungsvorrichtung gemäß dem Patentanspruch 1 gelöst.

Die Erfindung betrifft eine Vorrichtung zur Verabreichung eines fluiden Produkts, die ein Gehäuse mit einer Aufnahme für das Produkt, eine Fördereinrichtung zur Förderung des Produkts und eine Dosiereinrichtung für die Einstellung einer zu verabreichenden Produktdosis und Anzeigen der eingestellten Produktdosis umfasst. Das Gehäuse bildet eine Aufnahme für das Produkt, vorzugsweise eine Aufnahme für ein mit dem Produkt gefülltes Behältnis. Grundsätzlich könnte die Aufnahme jedoch auch selbst unmittelbar das Produktbehältnis bilden. Die Fördereinrichtung umfasst ein erstes Förderglied, das relativ zu dem Gehäuse in eine Förderrichtung bewegbar ist, um die eingestellte Produktdosis in einem entsprechend der Produktdosis voreingestellten Förderhub auszuschütten. Bei dem Förderhub handelt es sich vorzugsweise um eine translatorische Bewegung des ersten Förderglieds in die Förderrichtung, vorzugsweise um eine Linearbewegung entlang einer Förderachse. Das erste Förderglied kann insbesondere als Kolbenstange gebildet sein, die in solch einer Ausbildung mit einem Kolben der Fördereinrichtung verbunden ist, beispielsweise mit dem Kolben in einem Stück gebildet oder mit dem Kolben verschraubt oder verschnappt ist, oder bei dem Förderhub lose gegen eine Rückseite des Kolbens drückt. Die Fördereinrichtung umfasst ferner ein zweites Förderglied, das mit dem ersten Förderglied in einem ersten Gewindeeingriff ist, in dem es für die Einstellung der Produktdosis relativ zu dem ersten Förderglied und dem Gehäuse drehbar und für die Ausschüttung der eingestellten Produktdosis relativ zu dem Gehäuse in die Förderrichtung translatorisch bewegbar ist. Falls die Fördereinrichtung für die Förderung des Produkts einen axial bewegbaren Kolben aufweist, wie dies bevorzugt wird, kann das erste Förderglied insbesondere eine Kolbenstange bilden. In derartigen Ausfuhrungen kann das erste Förderglied grundsätzlich aber auch nur ein Übertragungsglied sein, das eine Förderbewegung des zweiten Förderglieds erst auf eine Kolbenstange überträgt.

Die Anzeigetrommel ist in einem zweiten Gewindeeingriff relativ zu dem Gehäuse drehbar sowie in und gegen die Förderrichtung translatorisch bewegbar. Vorzugsweise ist die Anzeigetrommel mit dem Gehäuse oder einer mit dem Gehäuse verbundenen Zwischenstruktur in dem zweiten Gewindeeingriff. Falls der zweite Gewindeeingriff mit einer Zwischenstruktur gebildet ist, ist die Zwischenstruktur mit dem Gehäuse vorzugsweise so verbunden, dass sie sich entweder bei der Einstellung der Dosis oder bei der Förderbewegung oder vorzugsweise in beiden Fällen relativ zu dem Gehäuse nicht bewegt.

Die Vorrichtung umfasst ferner eine Kupplung, welche die Anzeigetrommel mit dem zweiten Förderglied verdrehgesichert koppelt, wobei der für die verdrehsichere Kopplung von der Kupplung gebildete Kupplungseingriff sich automatisch löst, wenn die Vorrichtung für die Verabreichung betätigt wird. Vorteilhafterweise wird der Kupplungseingriff gelöst bevor der Förderhub des zweiten Förderglieds einsetzt. Indem das zweite Förderglied relativ zu dem Gehäuse in und gegen die Förderrichtung bewegbar ist, mit dem ersten Förderglied einen ersten Spindeltrieb bildet und während der Einstellung der Produktdosis über einen lösbaren Kupplungseingriff mit der Anzeigetrommel gekoppelt ist, können die Gewindesteigungen der beiden Spindeltriebe, nämlich der mit dem zweiten Förderglied gebildete erste Spindeltrieb und der mit der Anzeigetrommel gebildete zweite Spindeltrieb, zueinander auf einfache Weise in weiten Grenzen variiert werden. So kann der mit der Anzeigetrommel gebildete Spindeltrieb insbesondere eine große Gewindesteigung und der mit dem zweiten Förderglied gebildete Spindeltrieb eine besonders kleine Gewindesteigung aufweisen. Es kann somit ein kleiner Förderhub mit einem großen Anzeigehub kombiniert werden. So kann die Gewindesteigung im zweiten Gewindeeingriff beispielsweise drei-, vier oder fünfmal so groß sein wie die Gewindesteigung im ersten Gewindeeingriff.

Der Gewindeeingriff der Förderglieder ist vorzugsweise selbsthemmend, was vorteilhafterweise dadurch erreicht wird, dass die Steigung der im Gewindeeingriff befindlichen Gewinde ausreichend klein ist. Stattdessen oder zusätzlich kann das zweite Förderglied durch einen lösbaren Rasteingriff an einer unerwünschten Drehbewegung während des Förderhubs gehindert werden. Der Winkelabstand der Drehrastpositionen solch eines Rasteingriffs entspricht der Vergrößerung oder Verkleinerung der Dosis um eine einstellbare Einheit.

In bevorzugten Ausführungen wird die Kupplung mittels einer Feder in den Kupplungseingriff gespannt und umfasst hierfür ein erstes Kupplungsglied und ein zweites Kupplungsglied sowie die Feder, welche die Kupplungsglieder miteinander in den Kupplungseingriff spannt. Zumindest in Ausgestaltungen, in denen die für die Ausführung des Förderhubs erforderliche Kraft manuell aufgebracht wird, wird es bevorzugt, wenn der Kupplungseingriff durch die für den Förderhub aufzubringende Kraft gelöst wird. Vorteilhafterweise wird die den Förderhub bewirkende Betätigungskraft in die Förderrichtung ausgeübt, und die Feder spannt eines der Kupplungsglieder in oder vorzugsweise gegen die Förderrichtung in den Kupplungseingriff mit dem anderen. Der Kupplungseingriff, d.h. die durch die Kupplungsglieder hergestellte Verdrehsicherung, kann rein auf einem Reib Schluss beruhen, vorzugsweise beruht sie jedoch auf Formschluss oder umfasst zumindest eine Formschlussverbindung der Kupplungsglieder. Der Kupplungseingriff wird vorzugsweise durch eine in die Förderrichtung erfolgende Bewegung, die eines der Kupplungsglieder relativ zu dem anderen ausführt, gelöst. Vorteilhafterweise ist eines der Kupplungsglieder relativ zu dem anderen in die Förderrichtung längs der Förderachse aus dem Kupplungseingriff bewegbar.

Für die Betätigung umfasst die Vorrichtung ein Betätigungselement, durch dessen Betätigung der Kupplungseingriff gelöst wird. Wenigstens eines aus Anzeigetrommel und zweitem Förderglied, vorzugsweise die Anzeigetrommel und noch bevorzugter auch das zweite Förderglied, ist relativ zu dem Betätigungselement drehbar. Das Betätigungselement ist vorzugsweise permanent mit einem der beiden Kupplungsglieder verbunden. Es ist in und gegen eine Richtung, in die das betreffende Kupplungsglied aus dem Kupplungseingriff bewegt wird, nicht beweglich mit dem betreffenden Kupplungsglied verbunden. Bei der Betätigung gelangt das Betätigungselement vorzugsweise gegen das andere der Kupplungsglieder in einen Kontakt. Das Betätigungselement und das andere der Kupplungsglieder bilden für den Kontakt vorzugsweise reibarme Kontaktflächen um einer im Betätigungskontakt möglicherweise auftretenden Relativdrehung nur geringe Reibungskräfte entgegenzusetzen.

Die Feder der Kupplung ist vorzugsweise eine mechanische Feder. Sie wirkt vorteilhafterweise als Druckfeder. Alternativ kann sie aber beispielsweise auch als Zugfeder wirken, welche die Kupplungsglieder mit ihrer Federkraft in den Kupplungseingriff zieht. Li der bevorzugten Ausbildung als Druckfeder wirkt sie zum einen gegen eines der Kupplungsglieder und zum anderen vorzugsweise gegen das zweite Förderglied. Als Druckfeder kann sie alternativ auch als Druckgasfeder, beispielsweise Druckluftfeder, gebildet sein.

Die Verabreichungsvorrichtung umfasst eine Ausgleichseinrichtung, die in Förderrichtung längenvariabel ist und das zweite Förderglied mit der Anzeigetrommel verdrehgesichert verbindet, wenn der Kupplungseingriff besteht. Die Ausgleichseinrichtung umfasst bevorzugterweise eine Feder, die so angeordnet ist, dass die Länge der Ausgleichseinrichtung nur gegen eine rückstellende Elastizitätskraft der Feder verringerbar ist. Die Feder ist vorzugsweise als mechanische Feder gebildet, könnte grundsätzlich aber auch eine pneumatische Feder sein. Das zu der Feder der Kupplung gesagte gilt für die Feder der Ausgleichseinrichtung ebenfalls. Die Feder kann insbesondere die im Zusammenhang mit der Kupplung beschriebene Feder sein. Obgleich weniger bevorzugt, kann die dem Längenausgleich dienende Feder aber auch zusätzlich zu der Feder der Kupplung vorgesehen sein.

Die Ausgleichseinrichtung umfasst zwei in Förderrichtung einander überlappende und relativ zueinander verdrehgesichert geführte Ausgleichsstrukturen. Die zwei Ausgleichsstrukturen sind unmittelbar aneinander verdrehgeführt. Bei bestehendem Kupplungseingriff ist eine der Ausgleichsstrukturen mit der Anzeigetrommel und die andere mit dem zweiten Förderglied verdrehgesichert verbunden. Die Ausgleichseinrichtung ist in bevorzugten Ausführungen als Teleskop gebildet. Die Ausgleichsstrukturell bilden in derartigen Ausführungen je einen in Förderrichtung erstreckten Teleskopabschnitt. Die Feder der Ausgleichseinrichtung stützt sich in vorteilhaften Ausbildungen in die Förderrichtung an der einen und gegen die Förderrichtung an der anderen der Ausgleichsstrukturen ab, so dass die Federkraft trachtet, die Ausgleichsstrukturen auseinander zu treiben. Bevorzugt weist die Ausgleichseinrichtung nur genau zwei in Förderrichtung relativ zueinander bewegbare Ausgleichsstrukturen auf. Die Ausgleichsstrukturen sind in sich in Förderrichtung vorteilhafterweise steif, könnten aber auch selbst in Förderrichtung eine Elastizität aufweisen, so dass sogar auf eine separate Feder verzichtet werden könnte. So könnte die Ausgleichseinrichtung durchaus als Federbalg gebildet sein, der gleichzeitig auch eines der Kupplungsglieder bilden kann.

In bevorzugter Ausführung umfasst die Vorrichtung eine Führung für eine verdrehgesicherte Linearführung des ersten Förderglieds in Förderrichtung. Auf ein im Förderstrang nachgeordnetes Förderglied, beispielsweise einen Kolben, kann von solch einem ersten Förderglied vorteilhafterweise kein Drehmoment übertragen werden.

Bevorzugterweise ist eine Rückhalteeinrichtung vorgesehen, die in einem Eingriff mit dem ersten Förderglied eine Bewegung des ersten Förderglieds gegen die Förderrichtung verhindert. Die Rückhalteeinrichtung und das erste Förderglied, soweit sein Zusammenwirken mit der Rückhalteeinrichtung betroffen ist, können insbesondere wie von Zahnstangenpens her bekannt gebildet sein. So kann das erste Förderglied beispielsweise als Zahnstange mit wenigstens einer Sägezahnreihe gebildet sein, in welche die Rückhalteeinrichtung mit einer federnd biegbaren Rückhaltezunge eingreifend eine Bewegung des ersten Förderglieds gegen die Förderrichtung verhindert, die Bewegung in die Förderrichtung jedoch zulässt.

Die Vorrichtung kann insbesondere ein Injektionsgerät sein, vorteilhafterweise für subkutane Verabreichungen mittels Injektionsnadel oder auch für nadellose Druckinjektoren. Das Injektionsgerät ist bevorzugt ein Injektionspen. Wie bereits in Verbindung mit der Rückhalteeinrichtung erwähnt, kann die Vorrichtung in der Art eines Zahnstangenpens gebildet sein, wobei das erste Förderglied allerdings als kombinierte Zahn-Gewinde-Stange gebildet ist oder zumindest einen entsprechenden Abschnitt aufweist. Aufgrund ihres einfachen Aufbaus und daher günstigen Preises bietet sich die Vorrichtung auch als Einweggerät an, das nach Entleerung des Produktbehältnisses entsorgt wird.

Bevorzugte Merkmale der Erfindung werden auch in den Unteransprüchen und deren Kombinationen beschrieben.

Ein Ausführungsbeispiel der Erfindung wird nachfolgend anhand von Figuren erläutert. An dem Ausführungsbeispiel offenbar werdende Merkmale bilden je einzeln und in jeder Merkmalskombination die Gegenstände der Ansprüche und auch die vorstehend beschriebenen Ausgestaltungen vorteilhaft weiter. Es zeigen:
- Figur 1: eine erfindungsgemäße Vorrichtung in einem Zustand der Nulldosis-Einstellung in einer Ansicht auf eine Dosisanzeige,
- Figur 2: die Vorrichtung in einem Zustand der Maximaldosis-Einstellung in der Ansicht auf die Dosisanzeige,
- Figur 3: die Vorrichtung im Zustand der Maximaldosis-Einstellung in einem Längsschnitt,
- Figur 4: die Vorrichtung im Zustand der Nulldosis-Einstellung im gleichen Längsschnitt und
- Figur 5: einen proximalen Abschnitt der Vorrichtung in einem Zustand der Maximaldosis-Einstellung in dem Längsschnitt der Figuren 3 und 4.

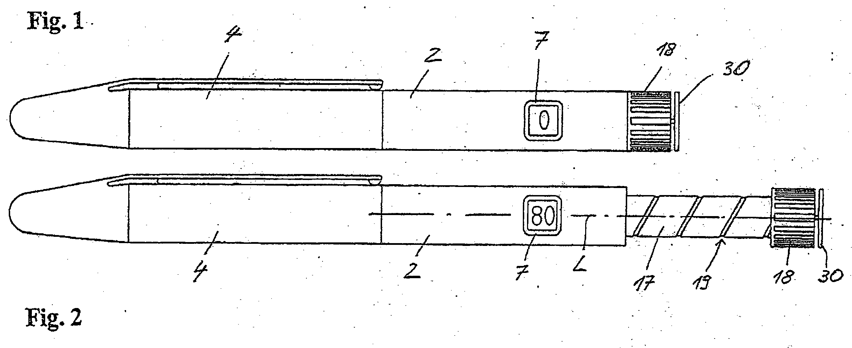

Figur 1 zeigt eine Vorrichtung für die dosierte Verabreichung eines fluiden Produkts, beispielsweise Insulin, ein Osteoporosepräparat oder ein Wachstumshormon. Die Vorrichtung ist als schlanker Injektionspen gebildet. Sie umfasst ein Gehäuse, von dem ein proximaler Gehäuseabschnitt 2 erkennbar ist. Eine Kappe 4 deckt einen distalen Gehäuseabschnitt ab. Die Vorrichtung befindet sich in einem Ausgangszustand, den sie vor Einstellung einer zu verabreichenden Produktdosis oder nach der Verabreichung einnimmt. Zu erkennen ist ein Dosierglied 18 für die Einstellung der Produktdosis und eine Dosisanzeige mit einem Fenster 7. Das Fenster 7 ist als Durchbrechung des Gehäuseabschnitts 2 gebildet und wird von einer durchsichtigen Abdeckung, beispielsweise eine Lupe abgedeckt; grundsätzlich kann das Fenster 7 auch einfach nur eine Durchbrechung sein, durch die auf einer Dosisskala die mittels des Dosierglieds 18 eingestellte Dosis ablesbar ist. Im Ausgangszustand der Figur 1 zeigt die Dosisanzeige die Dosis "Null" an. Ein tellerförmiges Betätigungselement 30 bildet ein proximales Ende der Vorrichtung.

Figur 2 zeigt die Vorrichtung in der gleichen Ansicht wie Figur 1, allerdings nach Einstellung einer mit der Vorrichtung pro Injektion maximal verabreichbaren Produktdosis. Wie an der Dosisanzeige ablesbar, entspricht die maximale Produktdosis beispielhaft 80 Dosiseinheiten des Produkts. Das Dosierglied 18 ist als Drehknopf gebildet. Durch eine Dosierdrehbewegung des Dosierglieds 18 wird die Dosis eingestellt. Durch die Dosierdrehbewegung wird eine Anzeigetrommel 17 längs einer zentralen Längsachse L der Vorrichtung aus dem Gehäuseabschnitt 2 herausgedreht. Die Dosisskala ist auf der äußeren Umfangsfläche der Anzeigetrommel 17 spiralig umlaufend aufgetragen. Die Anzeigetrommel 17 ist um die zentrale Längsachse L spiralig umlaufend mit einem Gewinde 19 versehen, das mit einem entsprechenden Innengewinde des Gehäuseabschnitts 2 in einem Gewindeeingriff steht, so dass die Anzeigetrommel 17 mit dem Gehäuseabschnitt 2 einen Spindeltrieb bildet. Die Steigung des Gewindeeingriffs bezüglich der zentralen Längsachse L ist so groß, dass eine Selbsthemmung nicht auftreten kann, wenn die Anzeigetrommel 17 durch Druck gegen das Betätigungselement 30 in den Gehäuseabschnitt 2 gedrückt wird.

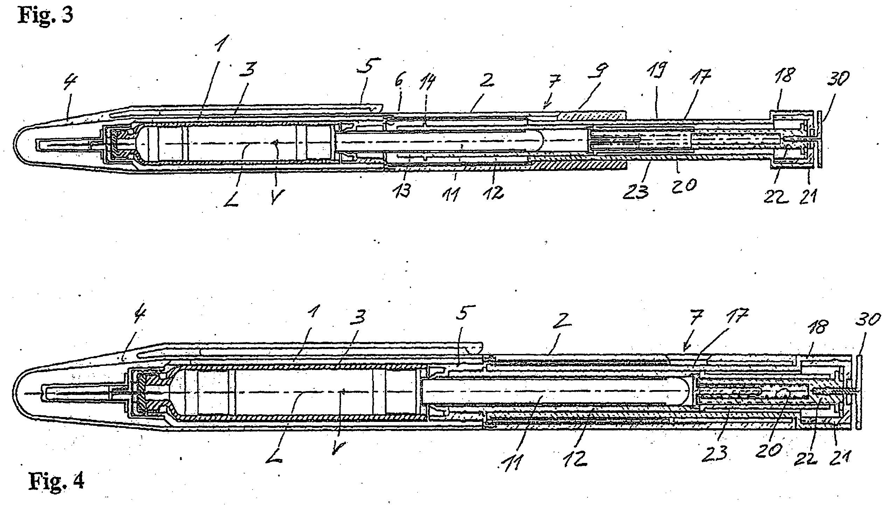

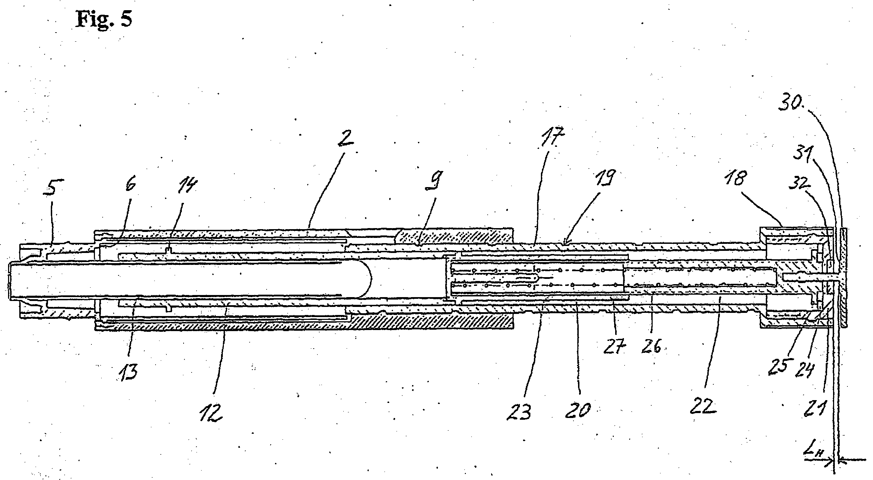

Figur 3 zeigt die Vorrichtung im Zustand der Figur 2, d.h. im Zustand der Maximaldosis-Einstellung, in einem Längsschnitt, der die zentrale Längsachse L der Vorrichtung enthält. Figur 4 zeigt die Vorrichtung im Zustand der Figur 1, d.h. in der Nulldosis-Einstellung, im gleichen Längsschnitt. Figur 5 zeigt wiederum im gleichen Längsschnitt, jedoch vergrößert, den proximalen Teil der Vorrichtung im Zustand der Maximaldosis-Einstellung.

Der distale Gehäuseabschnitt 1 und der damit unbeweglich verbundene proximale Gehäuseabschnitt 2 bilden ein Gehäuse 1, 2 der Vorrichtung. Der distale Gehäuseabschnitt 1 bildet eine Aufnahme für ein mit dem Produkt gefülltes Behältnis 3, das von einer Ampulle gebildet wird, wie sie beispielsweise aus der Diabetestherapie für Injektionspens bekannt ist. Ein distaler Auslass der Ampulle wird von einem Septum verschlossen. Eine längs der Längsachse L angeordnete Injektionskanüle durchragt das Septum. In dem Behältnis 3 ist ein Kolben längs der Längsrichtung L in eine Förderichtung V auf den Auslass zu bewegbar aufgenommen. Die Längsachse L bildet auch eine Förderachse der Vorrichtung und wird im folgenden auch so bezeichnet.

Eine Kolbenstange treibt den Kolben längs der Förderächse L in die Förderrichtung V. Die Kolbenstange bildet ein erstes Förderglied 11 einer Fördereinrichtung, die auch den Kolben und ferner ein das erste Förderglied 11 antreibendes zweites Förderglied 12 umfasst. Das erste Förderglied 11 ist in den Figuren 3, 4 und 5 nicht eingezeichnet, das Bezugszeichen "11" deutet auf den Einbauort. Das erste Förderglied 11 ist mit dem zweiten Förderglied 12 in einem Gewindeeingriff, wobei die Förderächse L die Gewindeachse ist. Das erste Förderglied 11 ist des weiteren mit einer Rückhalteeinrichtung 5 in einem Eingriff, die in und gegen die Förderrichtung V nicht beweglich mit dem Gehäuseabschnitt 2 verbunden ist. Die Rückhalteeinrichtung 5 bildet zwei Rückhaltezungen, die in Längsnuten des ersten Förderglieds 11 eingreifen und eine Bewegung des ersten Förderglieds 11 gegen der Förderrichtung V blockieren. Das erste Förderglied 11 ist für den Rückhalteeingriff in den beiden Nuten je mit einer Sägezahnreihe versehen. Das Zusammenwirken des ersten Förderglieds 1 und der Rückhalteeinrichtung 5 entspricht dem bekannter Zahnstangenpens. Ein Beispiel eines derartigen Pens wird in der DE 102 32 411 A1 beschrieben, auf die diesbezüglich verwiesen sei.

Das erste Förderglied 11 ist des weiteren relativ zu dem Gehäüseabschnitt 2 um die Förderachse L nicht drehbar, wobei eine hierfür gebildete Verdreh Sicherung eine Drehbewegung in beide Drehrichtungen in allen Betriebszuständen der Vorrichtung blockiert. Die Rückhalteeinrichtung 5 kann gleichzeitig auch die Verdrehsicherung bilden, eine Verdrehsicherung kann jedoch auch zusätzlich vorgesehen sein.

Das zweite Förderglied 12 ist hülsenförmig, umgibt das erste Förderglied 11 und weist an seinem distalen Ende ein Innengewinde 13 auf, das mit einem Außengewinde des ersten Förderglieds 11 in dem Gewindeeingriff ist. Das Innengewinde 13 und das Außengewinde des ersten Förderglieds 11 sind Feingewinde mit einer entsprechend geringen Gewindesteigung. Das zweite Förderglied 12 bildet ebenfalls noch im distalen Endbereich einen nach radial außen abragenden Förderanschlag 14, der im Zusammenwirken mit einem Gegenanschlag 6, den die Rückhalteeinrichtung 5 bildet, die Förderbewegung des zweiten Förderglieds 12 und damit wegen des Gewindeeingriffs auch des ersten Förderglieds 11 in die Förderrichtung V begrenzt. Den Anschlag 14 kann ein umlaufender Flansch oder eine Mehrzahl einzelner Nocken oder gegebenenfalls auch nur ein einzelner Nocken bilden, falls der Gegenanschlag 6 um die Förderachse L umläuft.

Über den Gewindeeingriff der Förderglieder 11 und 12 hinaus ist als weiterer, zweiter Gewindeeingriff derjenige zwischen der Anzeigetrommel 17 und dem Gehäuseabschnitt 2 gebildet, nämlich zwischen dem Außengewinde 19 der Anzeigetrommel 17 und einem Innengewinde 9 des Gehäuseabschnitts 2. Die Förderachse L ist auch die Gewindeachse des zweiten Gewindeeingriffs. Zwischen dem zweiten Förderglied 12 und dem Gehäuseabschnitt 2 verbleibt ein Ringspalt, in den die Anzeigetrommel 17 im zweiten Gewindeeingriff bei der Ausschüttung des Produkts einfährt und aus dem sie bei der Einstellung der Dosis ausfährt. Beim Ein- und Ausfahren gleitet sie über die von dem Anschlag 14 abgesehen glatte Mantelaußenfläche des zweiten Förderglieds 12. Die Anzeigetrommel 17 ist somit radial beidseitig geführt, zum einen im zweiten Gewindeeingriff 9, 19 und zum anderen durch das zweite Förderglied 12. Indem die Anzeigetrommel 17 das zweite Förderglied 12 eng anliegend umgibt, kann der Pen sehr schlank gehalten werden.

Das zweite Förderglied 12 ist für die Einstellung der Dosis über eine Kupplung verdrehgesichert mit dem Dosierglied 18 verbunden. Das Dosierglied 18 und die Anzeigetrommel 17 wiederum sind in einem Stück als Hülsenkörper gebildet mit der Anzeigetrommel 17 als langgestreckter distaler Hülsenabschnitt und dem Dosierglied 18 als demgegenüber breiterer, aber axial kürzerer proximaler Endabschnitt.

Die Kupplung umfasst ein erstes Kupplungsglied 21, ein zweites Kupplungsglied 22 und eine Feder 20, die als Spiralfeder gebildet ist und als Druckfeder wirkend das zweite Kupplungsglied 22 in einen Kupplungseingriff mit dem ersten Kupplungsglied 21 drückt. Im Kupplungseingriff sind die Kupplungsglieder 21 und 22 formschlüssig bezüglich der Förderachse L verdrehgesichert miteinander verbunden. Das erste Kupplungsglied 21 ist in den das Dosierglied 18 bildenden Hülsenabschnitt des Hülsenkörpers 17, 18 eingesetzt und mit dem Hülsenkörper 17, 18 sowohl verdrehgesichert als auch axial unbeweglich verbunden. Das erste Kupplungsglied 21 ist topfförmig mit einem Boden am proximalen Ende und einer von dem Boden umlaufend in die distale Richtung aufragenden Wand geformt. Es schließt somit den Hülsenkörper 17, 18 an dessen proximalen Ende ab. Für den Kupplungseingriff sind das erste Kupplungsglied 21 mit Kupplungsrippen, -nocken, -nuten 24 oder dergleichen und das zweite Kupplungsglied 22 mit entsprechenden Gegennuten, -rippen oder -nocken 25 oder dergleichen ausgestattet, die ineinandergreifend eine Drehbewegung eines der Kupplungsglieder 21 und 22 relativ zu dem anderen verhindern. Grundsätzlich genügt für den Kupplungseingriff natürlich auch nur ein einziges Paar aus beispielsweise Kupplungsrippe 24 und Kupplungsnut 25 als Eingriffselemente.

Das zweite Kupplungsglied 22 ist in Doppelfunktion auch Bestandteil einer Ausgleichseinrichtung, welche das Kupplungsglied 22 als eine erste Ausgleichsstruktur und eine damit zusammenwirkende zweite Ausgleichsstruktur 23 umfasst. Auch die Feder 20 erfüllt eine Doppelfunktion, zum einen als Kupplungsfeder der Kupplung und zum anderen als Ausgleichsfeder der Ausgleichseinrichtung. Die beiden Ausgleichsstrukturen 22 und 23 bilden ein Teleskop. Sie sind in und gegen die Förderrichtung V längs der Förderachse L aneinander verdrehgesichert linear geführt, wobei die Ausgleichsstruktur 22 einen inneren und die Ausgleichsstruktur 23 einen äußeren Teleskopabschnitt bildet. Für die Verdrehsicherung ist die Ausgleichsstruktur 22 an ihrer Mantelaußenfläche mit Führungselementen 26 und die Ausgleichsstruktur 23 an ihrer Mantelinnenfläche mit Führungsgegenelementen 27 versehen. Die Führungselemente 26 und 27 sind in der Art einer Nut- und Federführung gebildet. Im Grunde könnte die Führung auch mittels nur einem einzigen Führungselement und einem einzigen Führungsgegenelement gebildet sein. Wie die Figuren 3 bis 5 erkennen lassen, überlappen die beiden Ausgleichstrukturen 22 und 23 einander im Zustand der Nulldosis-Einstellung nahezu vollständig, während die axiale Überlappung im Zustand der Maximaldosis-Einstellung minimal ist und gerade noch die verdrehgesicherte Verbindung zwischen der Anzeigetrommel 17 und dem Dosierglied 18 einerseits und dem zweiten Förderglied 12 andererseits gewährleistet. Die Ausgleichsstruktur 23 ist topfförmig mit einem Boden am distalen Ende und einer von dem Boden umlaufend in die proximale Richtung aufragenden Hülsenwand. Die Feder 20 stützt sich in die Förderrichtung V an dem Boden der Ausgleichsstruktur 23 ab. Von dem Boden ragt ferner zentral eine Axialführung für die Feder 20 auf. Die Feder 20 drückt die Ausgleichsstruktur 23 in Förderrichtung V auf Anschlag gegen das zweite Förderglied 12. Ferner sind das zweite Förderglied 12 und die Ausgleichsstruktur 23 miteinander in einem verdrehgesicherten Eingriff bezüglich der Förderachse L. Für den verdreh gesicherten Eingriff weist das zweite Förderglied 12 an seinem proximalen Ende an einer Mantelinnenfläche axial kurze Eingriffselemente auf, die mit an der Mantelaußenfläche der Ausgleichsstruktur 23 gebildeten Gegenelementen in dem verdrehgesicherten Eingriff sind. Die Ausgleichsstruktur 23 ist im Ausführungsbeispiel ein separates Bauteil, sie kann alternativ jedoch in einem Stück mit dem zweiten Förderglied 12 geformt sein. Die Formung als separates Bauteil ist fertigungstechnisch jedoch vorteilhaft.

Das Betätigungselement 30 bildet einen tellerförmigen Abschluss der Vorrichtung. Von der tellerförmigen Struktur ragt in Förderrichtung V ein stiftförmiger Verbindungsabschnitt durch den Boden des ersten Kupplungsglieds 21 in das Innere des Dosierglieds 18 hinein und ist dort in einem Bodenabschnitt des ebenfalls topfförmigen zweiten Kupplungsglieds 22 verankert, so dass das Kupplungsglied 22 und das Betätigungselement 30 in und gegen die Förderrichtung V relativ zueinander nicht beweglich sind. Vorzugsweise sind sie relativ zueinander um die Förderachse L drehbar; grundsätzlich können sie jedoch auch verdrehgesichert miteinander verbunden sein. Das Betätigungselement 30 ist gemeinsam mit dem zweiten Kupplungsglied 22 gegen die rückstellende Elastizitätskraft der Feder 20 relativ zu dem ersten Kupplungsglied 21 in die Förderrichtung V bewegbar. Ferner ist es zumindest relativ zu dem ersten Kupplungsglied 21 um die Förderachse L drehbar.

Wie am besten in Figur 5 zu erkennen ist, verbleibt von dem Verbindungsabschnitt des Betätigungselements 30 abgesehen zwischen dem Betätigungselement 30 und dem ersten Kupplungsglied 21 axial ein lichter Abstand L_{H}. Der Abstand L_{H} besteht zwischen einander axial gegenüberliegenden Kontaktflächen des Betätigungselements 30 und des Kupplungsglieds 21. Die Kontaktfläche des Betätigungselements 30 ist mit 31 bezeichnet und ist um den Verbindungsabschnitt des Betätigungselements 30 zentral als in die distale Richtung aufragende, erhabene Fläche gebildet. Zur Bildung der Kontaktgegenfläche ist in das Kupplungsglied 21 komplementär zur Kontaktfläche 31 ein Kontaktelement 32 eingesetzt. Die Kontaktfläche 31 und die Gegenfläche des Kontaktelements 32 sind besonders reibarm gebildet, beispielsweise aus Teflon.

Nachfolgend wird die Funktion der Vorrichtung bei der Einstellung der Dosis und deren Verabreichung anhand der Figuren 3 und 4 beschrieben, wobei stets auch auf Figur 5 verwiesen sei:
Die Vorrichtung gelangt im Zustand der Nulldosis-Einstellung, wie Figur 4 ihn zeigt, zum Benutzer. In der Aufnahme des distalen Gehäuseabschnitts 1 ist das volle Behältnis 3 eingesetzt. Für eine erste Injektion zieht der Benutzer die Kappe 4 ab und entfernt einen die Injektionsnadel umgebenden Nadelschutz.

Vor Einstellung der gewünschten Dosis kann aus Sicherheitsgründen wieder die Kappe 4 aufgesteckt werden. Zum Einstellen der Dosis hält der Benutzer die Vorrichtung im Bereich des Gehäuses 1, 2 oder bei aufgesetzter Kappe 4 jedenfalls im Bereich des Gehäuseabschnitts 2 und verdreht das Dosierglied 18, bis die gewünschte Dosis eingestellt ist. Aufgrund der Dosierdrehbewegung fährt die Trommel 17 im zweiten Gewindeeingriff 9, 19 in die proximale Richtung aus dem Gehäuseabschnitt 2 heraus. Im Fenster 7 ist die der momentanen Dosierposition entsprechende Dosis ablesbar. Die Dosierdrehbewegung erfolgt in diskreten Schritten zwischen Rastpositionen. Dabei ist bei jedem Rastvorgang ein deutliches Klickgeräusch wahrnehmbar. Die Maximaldosis-Einstellung wird durch einen Anschlag der Anzeigetrommel 17 und einen Gegenanschlag des Gehäuseabschnitts 2 bestimmt. Zwischen den beiden Extremeinstellungen der Nulldosis und der Maximaldosis kann das Dosierglied 18 und damit gemeinsam die Anzeigetrommel 17 in beide Drehrichtungen beliebig von einer Rastposition in die nächste verdreht werden, so dass Dosiskorrekturen ohne weiteres möglich sind.

Bei dem Ausfahren von Anzeigetrommel 17 und Dosierglied 18 wird das zweite Kupplungsglied 22 von der Feder 20 permanent im Kupplungseingriff mit dem ersten Kupplungsglied 21 gehalten. Ferner ist das Kupplungsglied 22 während der Dosierdrehbewegung permanent im verdrehgesicherten Eingriff mit der Ausgleichsstruktur 23, die ihrerseits von der Feder 20 im verdrehgesicherten Eingriff mit dem zweiten Förderglied 12 gehalten wird. Der rotatorische Anteil der Dosierdrehbewegung wird somit auf das zweite Förderglied 12 übertragen. Da das erste Förderglied 11 relativ zu dem Gehäuse 1, 2 nicht drehbar ist und ferner von der Rückhalteeinrichtung 5 an einer Bewegung gegen die Förderrichtung V gehindert wird, schraubt sich das zweite Förderglied 12 im Gewindeeingriff mit dem ersten Förderglied 11 relativ zu diesem gegen die Förderrichtung V. Bei diesem Dosierhub, den das zweite Förderglied 12 relativ zu dem ersten Förderglied 11 ausführt, verändert sich der axiale Abstand zwischen dem Anschlag 14 und dem Gegenanschlag 6. Dieser axiale Abstand entspricht der Länge des Dosierhubs und entsprechend auch der Länge des Förderhubs.

Da die Gewindesteigung im zweiten Gewindeeingriff 9, 19 größer ist als im Gewindeeingriff der Förderglieder 11 und 12, im ausgeführten Gerät etwas mehr als viermal so groß, absolviert die Anzeigetrommel 17 bei der Dosiseinstellung bei gleichem Drehwinkel einen entsprechend dem Verhältnis der Steigungen längeren Hub. Entsprechend groß und ausreichend voneinander beabstandet sind die Dosiswerte auf der Dosisskala.

Der axiale Dosierhub der Anzeigetrommel 17 und des Dosierglieds 18, d.h. der translatorische Anteil der Dosierbewegung, ist länger als der Dosierhub des zweiten Förderglieds 12. Die Ausgleichseinrichtung 20, 22 und 23 gleicht die Längendifferenz aus, so dass im Kupplungseingriff zwischen den durch Anschläge begrenzten Extremeinstellungen, der Nulldosis-Einstellung der Maximaldosis-Einstellung, stets die verdrehgesicherte Verbindung zwischen der Anzeigetrommel 17 und dem Dosierglied 18 einerseits und dem zweiten Förderglied 12 andererseits besteht.

Nachdem die gewünschte Dosis eingestellt ist, beispielsweise die Maximaldosis, die im Ausführungsbeispiel 80 Einheiten beträgt (Figur 2), zieht der Benutzer die Kappe 4 ab, falls diese aus Sicherheitsgründen noch aufgesteckt war, sticht die Injektionsnadel an der gewünschten Einstechstelle durch die Haut und platziert die Nadelspitze so im subkutanen Gewebe.

Bei subkutan platzierter Injektionsnadel hält der Benutzer die Vorrichtung mit einer Hand im Bereich des Gehäuses 1, 2 und drückt mit dem Daumen in Förderrichtung V gegen das Betätigungselement 30. Durch den Druck wird in einer ersten Phase des Bestätigungshubs das zweite Kupplungsglied 22 gegen den Druck der Feder 20 relativ zu dem ersten Kupplungsglied 21 in Förderrichtung V aus dem Kupplungseingriff bewegt. Die axiale Länge der den Kupplungseingriff vermittelnden Eingriffselemente 24 und 25 ist so bemessen, dass der Kupplungseingriff gelöst ist, wenn das Betätigungselement 30 relativ zu dem Kupplungsglied 21 den Leerhub LH zurückgelegt hat. Bei weiterem Druck auf das Betätigungselement 30 drückt dieses über das Kontaktelement 32 gegen das Kupplungsglied 22, so dass sich die Anzeigetrommel 17 in der zweiten Phase des Betätigungshubs im zweiten Gewindeeingriff 9, 19 in den Gehäuseabschnitt 2 schraubt. Das zweite Kupplungsglied 22 fährt dabei teleskopartig in der Ausgleichsstruktur 23 bis gegen einen axialen Anschlag, den die Ausgleichsstruktur 23 im Ausführungsbeispiel durch ihren Boden bildet. Sobald das Kupplungsglied 22 gegen die Ausgleichsstruktur 23 auf Anschlag gelangt, bewirkt der weitere Druck auf das Betätigungselement 30, dass nun in der letzten Phase des Betätigungshubs das zweite Förderglied 22 wegen der andrückenden Ausgleichsstruktur 23 mitgenommen wird und den Förderhub ausführt. Der Förderhub wird durch das Anschlagpaar 6, 14 begrenzt. Der Betätigungshub ist zwar funktional in Phasen gegliedert, wird vorteilhafterweise aber kontinuierlich ausgeführt.

Nach vollständiger Ausführung des Förderhubs wird der Druck von dem Betätigungselement 30 genommen, so dass die Feder 20 das Kupplungsglied 22 wieder in den Kupplungseingriff mit dem Kupplungsglied 21 drückt. Nachdem die Injektionsnadel aus dem Gewebe gezogen wurde, wird die Kappe 4 wieder aufgesteckt. Die Vorrichtung befindet sich nun wieder im Zustand der Figur 4, allerdings ohne die nur vor der erstmaligen Benutzung vorhandene Nadelschutzkappe. In diesem Zustand wird die Vorrichtung bis zur nächsten Injektion aufbewahrt.

### Bezugszeichen:

- 1: distaler Gehäuseabschnitt
- 2: proximaler Gehäuseabschnitt
- 3: Behältnis
- 4: Kappe
- 5: Rückhalteeinrichtung, Verdrehsicherung
- 6: Förderanschlag
- 7: Fenster
- 8: -
- 9: Gewinde
- 10: -
- 11: erstes Förderglied
- 12: zweites Förderglied
- 13: Gewinde
- 14: Förderanschlag
- 15: -
- 16: -
- 17: Anzeigetrommel
- 18: Dosierglied
- 19: Gewinde
- 20: Feder
- 21: erstes Kupplungsglied
- 22: zweites Kupplungsglied, Ausgleichsstruktur
- 23: Ausgleichsstruktur
- 24: Eingriffselemente, Rippen
- 25: Eingriffselemente, Nuten
- 26: Führungselemente, Rippen
- 27: Führungselemente, Nuten
- 28: -
- 29: -
- 30: Betätigungselement

- 31: Kontaktflächen
- 32: Kontaktelement

- V: Förderrichtung, Betätigungsrichtung
- L: Längsachse, Förderachse

## Patentansprüche

1. Verabreichungsvorrichtung mit Anzeigetrommel, umfassend:
a) ein Gehäuse (1, 2) mit einer Aufnahme für ein zu verabreichendes Produkt,
b) ein erstes Förderglied (11), das für die Förderung des Produkts relativ zu dem Gehäuse (1, 2) in eine Förderrichtung (V) bewegbar ist,
c) ein zweites Förderglied (12), das in einem ersten Gewindeeingriff mit dem ersten Förderglied (11) relativ zu dem Gehäuse (1, 2) in und gegen die Förderrichtung (V), relativ zu dem Gehäuse (1, 2) und dem ersten Förderglied (11) rotatorisch und relativ zu dem ersten Förderglied (11) gegen die Förderrichtung (V) translatorisch bewegbar ist, wobei das zweite Förderglied (12) hülsenförmig ist und an seinem distalen Ende ein Innengewinde (13) aufweist, welches mit einem Außengewinde des ersten Förderglieds (11) in dem ersten Gewindeeingriff ist,
d) eine Dosiereinrichtung, mittels der eine Produktdosis einstellbar ist und die für die Anzeige der Produktdosis die Anzeigetrommel (17) umfasst, die in einem zweiten Gewindeeingriff relativ zu dem Gehäuse (1, 2) rotatorisch und in und gegen die Förderrichtung (V) translatorisch bewegbar ist,
e) eine ein erstes Kupplungsglied (21) und ein zweites Kupplungsglied (22) umfassende Kupplung (20-22), die das zweite Förderglied (12) und die Anzeigetrommel (17) in einem Kupplungseingriff verdrehgesichert miteinander verbindet, wobei der Kupplungseingriff durch Betätigung eines Betätigungselements (30) lösbar ist, wobei das Betätigungselement (30) als ein von dem zweiten Kupplungsglied (21, 22) separates Teil in und gegen eine Richtung, in die das zweite Kupplungsglied (21, 22) aus dem Kupplungseingriff bewegt wird, nicht beweglich mit dem zweiten Kupplungsglied (21, 22) verbunden ist und
f) eine Ausgleichseinrichtung (22, 23), die in Förderrichtung (V) längenvariabel ist und bei bestehendem Kupplungseingriff das zweite Förderglied (12) mit der Anzeigetrommel (17) verdrehgesichert verbindet,
**dadurch gekennzeichnet, dass**
g) die Ausgleichseinrichtung (22, 23) eine erste Ausgleichsstruktur und eine zweite Ausgleichsstruktur (23) umfasst, die in Vortriebsrichtung (V) einander überlappen und relativ zueinander und unmittelbar aneinander verdrehgesichert geführt sind und wobei bei bestehendem Kupplungseingriff eine der Ausgleichsstrukturen mit der Anzeigetrommel (17) und die andere mit dem zweiten Förderglied (12) verdrehgesichert verbunden ist, wobei das zweite Kupplungsglied (22) die erste Ausgleichsstruktur bildet und die zweite Ausgleichsstruktur (23) in einem Stück mit dem zweiten Förderglied (12) geformt ist.

2. Verabreichungsvorrichtung nach Anspruch 1, **dadurch gekennzeichnet, dass** wenigstens eines aus Anzeigetrommel (17) und zweitem Förderglied (12) relativ zu dem Betätigungselement (30) drehbar ist.

3. Verabreichungsvorrichtung nach einem der vorhergehenden Ansprüche, **dadurch gekennzeichnet, dass** die Kupplung (20-22) eine das zweite Kupplungsglied (22) in den Kupplungseingriff mit dem ersten Kupplungsglied (21) spannende Feder (20) umfasst.

4. Verabreichungsvorrichtung nach den Ansprüchen 2 und 3, **dadurch gekennzeichnet, dass** das Betätigungselement (30) in und gegen eine Richtung, in die das zweite Kupplungsglied (22) aus dem Kupplungseingriff bewegt wird, nicht beweglich mit dem zweiten Kupplungsglied (22) verbunden ist.

5. Verabreichungsvorrichtung nach einem der zwei vorhergehenden Ansprüche, **dadurch gekennzeichnet, dass** das Betätigungselement (30) permanent mit dem zweiten Kupplungsglied (22) verbunden ist.

6. Verabreichungsvorrichtung nach Anspruch 3, **dadurch gekennzeichnet, dass** die Anzeigetrommel (17) mit einem der Kupplungsglieder (21, 22) verdrehgesichert verbunden ist oder das eine der Kupplungsglieder (21, 22) bildet.

7. Verabreichungsvorrichtung nach einem der Ansprüche 3 und 6, **dadurch gekennzeichnet, dass** die Feder (20) das zweite Kupplungsglied (22) in oder vorzugsweise gegen die Förderrichtung (V) in den Kupplungseingriff spannt.

8. Verabreichungsvorrichtung nach einem der Ansprüche 3, 6 und 7, **dadurch gekennzeichnet, dass** das zweite Förderglied (12) und das zweite Kupplungsglied (22) längs einer gemeinsamen Achse (L) translatorisch bewegbar sind.

9. Verabreichungsvorrichtung nach einem der vorhergehenden Ansprüche, **dadurch gekennzeichnet, dass** der Kupplungseingriff formschlüssig ist.

10. Verabreichungsvorrichtung nach Anspruch 2 in Kombination mit Anspruch 3, **dadurch gekennzeichnet, dass** das Betätigungselement (30) für die Betätigung des zweiten Förderglieds (12) relativ zu wenigstens einem aus Gehäuse (1, 2) und erstem Kupplungsglied (21) bewegbar ist und mit dem zweiten Kupplungsglied (22) so gekoppelt ist, dass es bei einer durch die Betätigung bewirkten Bewegung, die vorzugsweise in die Förderrichtung (V) stattfindet, das zweite Kupplungsglied (22) aus dem Kupplungseingriff bewegt.

11. Verabreichungsvorrichtung nach dem vorhergehenden Anspruch, **dadurch gekennzeichnet, dass** das Betätigungselement (30) nach dem Lösen des Kupplungseingriffs in einen Eingriff gelangt, in dem es bei weiterer Bewegung die Anzeigetrommel (17) mitnimmt, wobei bevorzugt ist, dass das Betätigungselement (30) in den Eingriff mit der Anzeigetrommel (17) erst gelangt, nachdem der Kupplungseingriff gelöst ist.

12. Verabreichungsvorrichtung nach einem der zwei vorhergehenden Ansprüche, **dadurch gekennzeichnet, dass** das Betätigungselement (30) nach dem Lösen des Kupplungseingriffs über das zweite Kupplungsglied (22) in eine Wirkverbindung mit dem zweiten Förderglied (12) gelangt, in der es bei weiterer Bewegung das zweite Förderglied (12) mitnimmt.

13. Verabreichungsvorrichtung nach einem der vorhergehenden Ansprüche, **dadurch gekennzeichnet, dass** eine Feder (20) vorgesehen ist, gegen deren Federkraft die Länge der Ausgleichseinrichtung (20, 22, 23) verringerbar ist, und/oder die Ausgleichseinrichtung (20, 22, 23) ein Teleskop bildet.

14. Verabreichungsvorrichtung nach dem vorhergehenden Anspruch, **dadurch gekennzeichnet, dass** eine Feder (20) vorgesehen ist, die in die Förderrichtung (V) auf eine der Ausgleichsstrukturen (22, 23) und gegen die Förderrichtung (V) auf die andere der Ausgleichsstrukturen (22, 23) wirkt.

15. Verabreichungsvorrichtung nach den Ansprüchen 3 und 14, **dadurch gekennzeichnet, dass** die Feder (20) der Ausgleichseinrichtung (20, 22, 23) die Feder (20) der Kupplung (20-22) ist.

16. Verabreichungsvorrichtung nach einem der vorhergehenden Ansprüche, **dadurch gekennzeichnet, dass** eine Führung vorgesehen ist, mittels der das erste Förderglied (11) entlang einer in Vortriebsrichtung (V) weisenden Förderachse (L) bezüglich beider Drehrichtungen relativ zu dem Gehäuse (1, 2) nicht drehbar geführt wird oder führbar ist und/oder dass eine Rückhalteeinrichtung (5) vorgesehen ist, um in einem Eingriff mit dem ersten Förderglied (11) eine Bewegung des ersten Förderglieds (11) gegen die Förderrichtung (V) zu verhindern.

17. Verabreichungsvorrichtung nach einem der vorhergehenden Ansprüche, **dadurch gekennzeichnet, dass** das erste Förderglied (11) eine Kolbenstange ist.

## Claims

1. Administration device having a display drum, comprising:
a) a housing (1, 2) having a receiver for a product to be administered,
b) a first conveyor member (11) which is able to be moved in a conveying direction (V) relative to the housing (1, 2) for conveying the product,
c) a second conveyor member (12) which, in a first threaded engagement with the first conveyor member (11), is able to be moved in and against the conveying direction (V) relative to the housing (1, 2), rotationally relative to the housing (1, 2) and the first conveyor member (11) and translationally against the conveying direction (V) relative to the first conveyor member (11), wherein the second conveyor member (12) is sleeve-like and has an internal thread (13) on its distal end, said internal thread being in the first threaded engagement with an external thread of the first conveyor member (11),
d) a metering device, by means of which a product dose is able to be regulated and which comprises the display drum (17) for the display of the product dose, said display drum being able to be moved, in a second threaded engagement, rotationally relative to the housing (1, 2) and translationally in and against the conveying direction (V),
e) a coupling (20-22) comprising a first coupling member (21) and a second coupling member (22), said coupling connecting the second conveyor member (12) and the display drum (17) to each other in a coupling engagement in a non-rotatable manner, wherein the coupling engagement is able to be released by actuating an actuating element (30), wherein the actuating element (30) is connected non-moveably to the second coupling element (21, 22) as a part which is separate from the second coupling member (21, 22) in and against a direction in which the second coupling member (21, 22) is moved out of the coupling engagement, and
f) a compensating device (22, 23) which is variable in length in the conveying direction (V) and connects the second conveyor member (12) to the display drum (17) in a non-rotatable manner in the event of an existing coupling engagement,
**characterised in that**
g) the compensating device (22, 23) comprises a first compensating structure and a second compensating structure (23) which overlap each other in the driving direction (V) and are guided relative to each other and directly against each other in a non-rotatable manner and wherein one of the compensating structures is connected to the display drum (17) and the other is connected to the second conveyor member (12) in a non-rotatable manner in the event of an existing coupling engagement, wherein the second coupling member (22) forms the first compensating structure and the second compensating structure (23) is formed in a unit with the second conveyor member (12).

2. Administration device according to claim 1, **characterised in that** at least one of the display drum (17) and second conveyor member (12) is able to be rotated relative to the actuating element (30).

3. Administration device according to one of the preceding claims, **characterised in that** the coupling (20-22) comprises a spring (20) tensioning the second coupling member (22) in the coupling engagement with the first coupling member (21).

4. Administration device according to claims 2 and 3, **characterised in that** the actuating element (30) is connected non-moveably to the second coupling member (22) in and against a direction in which the second coupling member (22) is moved out of the coupling engagement.

5. Administration device according to one of the two preceding claims, **characterised in that** the actuating element (30) is permanently connected to the second coupling member (22).

6. Administration device according to claim 3, **characterised in that** the display drum (17) is connected to one of the coupling members (21, 22) in a non-rotatable manner or forms one of the coupling members (21, 22).

7. Administration device according to one of claims 3 and 6, **characterised in that** the spring (20) tensions the second coupling member (22) in or preferably against the conveying direction (V) in the coupling engagement.

8. Administration device according to one of claims 3, 6 and 7, **characterised in that** the second conveyor member (12) and the second coupling member (22) are able to be moved along a common axis (L) in a translational manner.

9. Administration device according to one of the preceding claims, **characterised in that** the coupling engagement is positive.

10. Administration device according to claim 2 in combination with claim 3, **characterised in that** the actuating element (30) is able to be moved relative to at least one of the housing (1, 2) and first coupling member (21) to actuate the second conveyor member (12), and is coupled to the second coupling member (22) in such a way that it moves the second coupling member (22) out of the coupling engagement in the event of a movement caused by actuation, said movement preferably taking place in the conveying direction (V).

11. Administration device according to the preceding claim, **characterised in that** the actuating element (30) enters into engagement after the release of the coupling engagement, in which engagement it picks up the display drum (17) in the event of further movement, wherein it is preferred that the actuating element (30) only enters into engagement with the display drum (17) after the coupling engagement is released.

12. Administration device according to one of the two preceding claims, **characterised in that** the actuating element (30) enters into an operative connection with the second conveyor member (12) after the release of the coupling engagement via the second coupling member (22), in which operative connection it takes up the second conveyor member (12) in the event of further movement.

13. Administration device according to one of the preceding claims, **characterised in that** a spring (20) is provided, against the spring force of which the length of the compensating device (20, 22, 23) is able to be reduced, and/or the compensating device (20, 22, 23) forms a telescope.

14. Administration device according to the preceding claim, **characterised in that** a spring (20) is provided which acts in the conveying direction (V) on one of the compensating structures (22, 23) and against the conveying direction (V) on the other of the compensating structures (22, 23).

15. Administration device according to claims 3 and 14, **characterised in that** the spring (20) of the compensating device (20, 22, 23) is the spring (20) of the coupling (20-22).

16. Administration device according to one of the preceding claims, **characterised in that** a guide is provided, by means of which the first conveyor member (11) is or is able to be guided non-rotatably along a conveying axis (L), pointing in the driving direction (V), with respect to both directions of rotation relative to the housing (1, 2), and/or a retaining means (5) is provided in order to prevent a movement of the first conveyor member (11) against the conveying direction (V) in engagement with the first conveyor member (11).

17. Administration device according to one of the preceding claims, **characterised in that** the first conveyor member (11) is a piston rod.

## Revendications

1. Dispositif d'administration comprenant un tambour d'affichage, comprenant :
a) un boîtier (1, 2) pourvu d'un logement pour un produit à administrer,
b) un premier organe de refoulement (11), qui peut être déplacé par rapport au boîtier (1,2) dans une direction de refoulement (V) en vue du refoulement du produit,
c) un deuxième organe de refoulement (12), qui, en prise par un filetage avec le premier organe de refoulement (11), peut être déplacé par rapport au boîtier (1, 2) dans la direction de refoulement (V) et dans le sens opposé à cette dernière, en rotation par rapport au boîtier (1, 2) et au premier organe de refoulement (11) et par translation par rapport au premier organe de refoulement (11) dans le sens opposé à la direction de refoulement (V), le deuxième organe de refoulement (12) ayant une forme de manchon et présentant, au niveau de son extrémité distale, un filetage intérieur (13), qui se trouve dans une première prise par filetage avec un filetage extérieur du premier organe de refoulement (11),
d) un système de dosage, au moyen duquel une dose de produit peut être réglée et qui comprend, en vue de l'affichage de la dose de produit, le tambour d'affichage (17), qui, dans une deuxième prise par filetage, peut être déplacé en rotation par rapport au boîtier (1, 2) et par translation dans la direction de refoulement (V) et dans le sens opposé à cette dernière,
e) un couplage (20 - 22) comprenant un premier organe de couplage (21) et un deuxième organe de couplage (22), lequel relie l'un à l'autre le deuxième organe de refoulement (12) et le tambour d'affichage (17) dans une prise par couplage de manière à empêcher toute torsion, la prise par couplage pouvant être déconnectée par l'actionnement d'un élément d'actionnement (30),
l'élément d'actionnement (30) en tant que pièce séparée du deuxième organe de couplage (21, 22) étant, aussi bien dans une direction dans laquelle le deuxième organe de couplage (21, 22) est déplacé de manière à sortir de la prise par couplage que dans le sens opposé à cette direction, en liaison non mobile avec le deuxième organe de couplage (21, 22) et
f) un système de compensation (22, 23), dont la longueur varie dans la direction de refoulement (V) et qui, en présence d'une prise par couplage, relie le deuxième organe de refoulement (12) au tambour d'affichage (17) de manière à empêcher toute torsion,
**caractérisé en ce**
g) **que** le système de compensation (22, 23) comprend une première structure de compensation et une deuxième structure de compensation (23), qui se chevauchent l'une l'autre dans une direction d'avancement (V) et qui sont guidées l'une par rapport à l'autre et directement l'une au niveau de l'autre de manière à empêcher toute torsion, une des structures de compensation étant reliée au tambour d'affichage (17) et l'autre étant reliée au deuxième organe de refoulement (12) de manière à empêcher toute torsion en présence d'une prise par couplage, le deuxième organe de couplage (22) formant la première structure de compensation et la deuxième structure de compensation (23) étant formée d'un seul tenant avec le deuxième organe de refoulement (12).

2. Dispositif d'administration selon la revendication 1, **caractérisé en ce qu'**au moins un élément parmi le tambour d'affichage (17) et le deuxième organe de refoulement (12) peut être tourné par rapport à l'élément d'actionnement (30).

3. Dispositif d'administration selon l'une quelconque des revendications précédentes, **caractérisé en ce que** le couplage (20 - 22) comprend un ressort (20) serrant le deuxième organe de couplage (22) dans la prise par couplage avec le premier organe de couplage (21).

4. Dispositif d'administration selon les revendications 2 et 3, **caractérisé en ce que**, aussi bien dans une direction dans laquelle le deuxième organe de couplage (22) est déplacé de manière à sortir de la prise par couplage qu'en sens opposé à cette direction, l'élément d'actionnement (30) est en liaison non mobile avec le deuxième organe de couplage (22).

5. Dispositif d'administration selon l'une quelconque des deux revendications précédentes, **caractérisé en ce que** l'élément d'actionnement (30) est relié de manière permanente au deuxième organe de couplage (22).

6. Dispositif d'administration selon la revendication 3, **caractérisé en ce que** le tambour d'affichage (17) est relié de manière à empêcher toute torsion avec l'un des organes de couplage (21, 22) ou forme l'un des organes de couplage (21, 22).

7. Dispositif d'administration selon l'une quelconque des revendications 3 et 6, **caractérisé en ce que** le ressort (20) serre dans la prise par couplage le deuxième organe de couplage (22) dans la direction de refoulement (V) ou de préférence dans le sens opposé à la direction de refoulement.

8. Dispositif d'administration selon l'une quelconque des revendications 3, 6 et 7, **caractérisé en ce que** le deuxième organe de refoulement (12) et le deuxième organe de couplage (22) peuvent être déplacés par translation le long d'un axe commun (L).

9. Dispositif d'administration selon l'une quelconque des revendications précédentes, **caractérisé en ce que** la prise par couplage est à complémentarité de forme.

10. Dispositif d'administration selon la revendication 2 en combinaison avec la revendication 3, **caractérisé en ce que** l'élément d'actionnement (30) peut être déplacé, en vue de l'actionnement du deuxième organe de refoulement (12), par rapport à au moins un élément parmi le boîtier (1, 2) et le premier organe de couplage (21) et est couplé au deuxième organe de couplage (22) de telle sorte qu'il déplace lors d'un déplacement provoqué par l'actionnement, qui a lieu de préférence dans la direction de refoulement (V), le deuxième organe de couplage (22) hors de la prise par couplage.

11. Dispositif d'administration selon la revendication précédente, **caractérisé en ce que** l'élément d'actionnement (30) parvient, après la déconnexion de la prise par couplage, dans une prise, dans laquelle il entraîne le tambour d'affichage (17) lors de la poursuite du déplacement, l'élément d'actionnement (30) parvenant de préférence dans la prise avec le tambour d'affichage (17) seulement après la déconnexion de la prise par couplage.

12. Dispositif d'administration selon l'une quelconque des deux revendications précédentes, **caractérisé en ce que** l'élément d'actionnement (30) parvient, après la déconnexion de la prise par couplage, par l'intermédiaire du deuxième organe de couplage (22), dans une liaison fonctionnelle avec le deuxième organe de refoulement (12), dans laquelle il entraîne le deuxième organe de refoulement (12) lors de la poursuite du déplacement.

13. Dispositif d'administration selon l'une quelconque des revendications précédentes, **caractérisé en ce qu'**est prévu un ressort (20), à l'encontre de la force de ressort duquel la longueur du système de compensation (20, 22, 23) peut être réduite, et/ou **en ce que** le système de compensation (20, 22, 23) forme un télescope.

14. Dispositif d'administration selon la revendication précédente, **caractérisé en ce qu'**est prévu un ressort (20), qui agit, dans la direction de refoulement (V), sur l'une des structures de compensation (22, 23) et, dans le sens opposé à la direction de refoulement (V), sur l'autre des structures de compensation (22, 23).

15. Dispositif d'administration selon les revendications 3 et 14, **caractérisé en ce que** le ressort (20) du système de compensation (20, 22, 23) est le ressort (20) du couplage (20 - 22).

16. Dispositif d'administration selon l'une quelconque des revendications précédentes, **caractérisé en ce qu'**est prévu un guidage, au moyen duquel le premier organe de refoulement (11) est guidé ou peut être guidé de manière à ne pas pouvoir tourner par rapport au boîtier (1, 2) le long d'un axe de refoulement (L) pointant dans une direction d'avancement (V) eu égard à deux directions de rotation, et/ou **en ce qu'**est prévu un système de retenue (5) afin d'empêcher, dans une prise avec le premier organe de refoulement (11), un déplacement du premier organe de refoulement (11) dans le sens opposé à la direction de refoulement (V).

17. Dispositif d'administration selon l'une quelconque des revendications précédentes, **caractérisé en ce que** le premier organe de refoulement (11) est une tige de piston.
